Europäisches Patentamt

European Patent Office

Office européen des brevets

(11) Numéro de publication : **0 339 693 B1**

(12) **FASCICULE DE BREVET EUROPEEN**

(45) Date de publication du fascicule du brevet :
**18.03.92 Bulletin 92/12**

(51) Int. Cl.$^5$ : **A61F 7/00**

(21) Numéro de dépôt : **89112085.9**

(22) Date de dépôt : **25.03.85**

(54) **Appareil d'examen et de localisation de tumeurs par ultrasons muni d'un dispositif de traitement localisé par hyperthermie.**

(30) Priorité : **03.05.84 FR 8406877**

(43) Date de publication de la demande :
**02.11.89 Bulletin 89/44**

(45) Mention de la délivrance du brevet :
**18.03.92 Bulletin 92/12**

(84) Etats contractants désignés :
**BE CH DE GB IT LI NL SE**

(56) Documents cités :
**EP-A- 0 068 961**
**EP-A- 0 081 639**
**DE-A- 2 722 252**
**GB-A- 2 126 901**
**US-A- 4 204 435**

(56) Documents cités :
**MESURES, REGULATION, AUTOMATISME,
vol. 45, février 1980, pages 25-29;
"Echographie ultrasonore: un circuit CCD
pour simplifier l'electronique de commande"
ULTRASONICS, vol. 5, avril 1967, pages
105-112; P.P. LELE: "Production of deep focal
lesions by focused ultrasound-current status"**

(60) Numéro de publication de la demande initiale
en application de l'article 76 CBE : **0 162 735**

(73) Titulaire : **Dory, Jacques
91 rue des Molveaux
F-77450 Coupvray Esblay (FR)**

(72) Inventeur : **Dory, Jacques
91 rue des Molveaux
F-77450 Coupvray Esblay (FR)**

(74) Mandataire : **Marquer, Francis et al
Cabinet Moutard 35, Avenue Victor Hugo
F-78960 Voisins le Bretonneux (FR)**

Jouve, 18, rue Saint-Denis, 75001 PARIS

## Description

Les appareils d'échographie classiques permettent évidemment de procéder à l'examen de tumeurs à l'intérieur du corps en formant une image sur l'écran d'un tube cathodique.

On sait par ailleurs qu'il est possible d'obtenir une destruction des cellules - en particulier des cellules malignes - en les soumettant de façon plus ou moins prolongée à une élévation de température. Les cellules à détruire doivent par exemple être portées à environ 45°C et ce, de manière bien contrôlée, en évitant d'atteindre des températures excessives qui pourraient provoquer des brûlures graves autour de la lésion. Le problème technique à résoudre consiste donc à la fois à maîtriser la quantité d'énergie et sa localisation.

Les différents procédés antérieurs (emploi d'hyperfréquences, de rayonnement infra-rouge, et autres) permettent de traiter des tumeurs superficielles, mais non d'atteindre des tissus plus profonds.

Selon EP-A-68 961 un dispositif d'hyperthermie combine un générateur d'un faisceau focalisé d'ultrasons comportant un émetteur principal d'ondes électriques à haute fréquence et un transducteur piézo-électrique principal, à un dispositif d'échographie comprenant un générateur auxiliaire d'impulsions électriques à haute fréquence associé à un transducteur piézo-électrique auxiliaire et à des moyens de formation d'images de la zone à traiter.

Ce brevet ne prévoit pas d'interrompre l'émission principale pour pouvoir effectuer l'échographie pendant le traitement lui-même.

Par ailleurs, la focalisation du faisceau principal y est obtenue par déphasage électronique, et aucun balayage échographique n'est prévu.

L'invention propose de réaliser un appareil qui conjugue les trois fonctions de localisation de la zone à traiter, de traitement par élévation de température bien maîtrisée dans une région restreinte bien délimitée à l'intérieur de cette zone et de contrôle simultané des résultats du traitement.

L'appareil de traitement par hyperthermie suivant l'invention combine un générateur d'un faisceau focalisé d'ultrasons comportant un émetteur principal d'ondes électriques à haute fréquence et un transducteur piézo-électrique principal, à un dispositif d'échographie comprenant un générateur auxiliaire d'impulsions électriques à haute fréquence associé à un transducteur piézo-électrique auxiliaire et à des moyens de formation d'images de la zone à traiter et est caractérisé en ce que la surface active du transducteur principal est focalisante, que des moyens sont prévus pour provoquer un balayage de formation d'images de la zone à traiter par le faisceau ultrasonore d'examen engendré par le transducteur auxiliaire et que des moyens de commutation et de réglage permettent de provoquer, pendant un mode principal de fonctionnement en traitement et contrôle, l'émission dudit faisceau focalisé par le transducteur principal excité par l'émetteur principal pendant des intervalles de temps périodiques séparés par des intervalles de temps plus petits pendant lesquels l'émission du faisceau d'examen et la formation d'images échographiques s'effectuent.

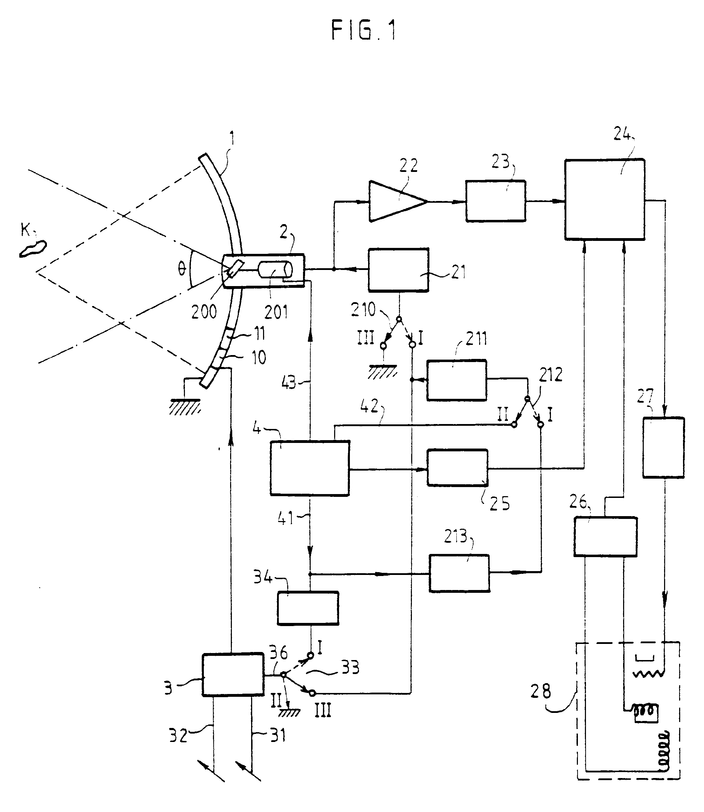

On notera que l'utilisation d'un transducteur à surface focalisante était connue en soi selon ULTRASONICS, vol. 5, avril 1967, figure 1c, tandis que le balayage échographique d'une zone à traiter était également connu en soi selon MESURES REGULATION AUTOMATISME, vol. 45, février 1980.

L'appareil comporte avantageusement un second mode de fonctionnement auxiliaire en repérage pendant lequel seule l'émission périodique du faisceau d'examen par le transducteur auxiliaire s'effectue et de préférence un troisième mode de fonctionnement auxiliaire en contrôle de la région focale, pendant lequel l'émission périodique du faisceau focalisé s'effectue, mais l'émetteur principal est synchronisé par le circuit de synchronisation du générateur auxiliaire pour fonctionner en échographie, les intervalles de temps qui séparent les périodes d'émission successives pendant les deux modes auxiliaires de fonctionnement étant sensiblement plus faibles que les intervalles qui séparent les périodes d'émission du faisceau focalisé pendant le mode principal.

Il résulte de ce qui précède que, pendant les modes auxiliaires de fonctionnement, destinés à effectuer des réglages précis, la qualité de l'image échographique, soit de la zone à traiter (mode de repérage), soit de la région focale (mode de contrôle de la région restreinte), sera sensiblement meilleure que pendant le mode de traitement, pendant lequel les images successives de la zone à traiter se succèderont par exemple à des intervalles de l'ordre de la seconde, ce qui permettra toutefois de vérifier de manière satisfaisante la position de la région focale pendant le traitement.

Suivant une forme d'exécution préférée, le transducteur principal est constitué par une mosaïque d'éléments piézo-électriques isolés les uns des autres et formant une calotte sphérique associée à des moyens de contrôler son déplacement suivant trois axes orthogonaux, tandis que le transducteur auxiliaire est solidarisé au sommet de ladite calotte et associé à des moyens d'effectuer un balayage sectoriel dans un plan qui passe par l'axe de symétrie de ladite calotte.

D'autres particularités, ainsi que les avantages de l'invention, apparaîtront clairement à la lumière de la description ci-après.

Au dessin annexé :

La figure 1 est le schéma de principe d'un appareil d'hyperthermie conforme à un mode d'exécution préféré de l'invention ;

La figure 2 représente schématiquement, en perspective, le transducteur principal et son dispositif de support mobile ;

La figure 3 représente les formes d'ondes en différents points des circuits de l'appareil ; et

La figure 4 illustre l'image obtenue sur l'écran de visualisation que comporte l'appareil.

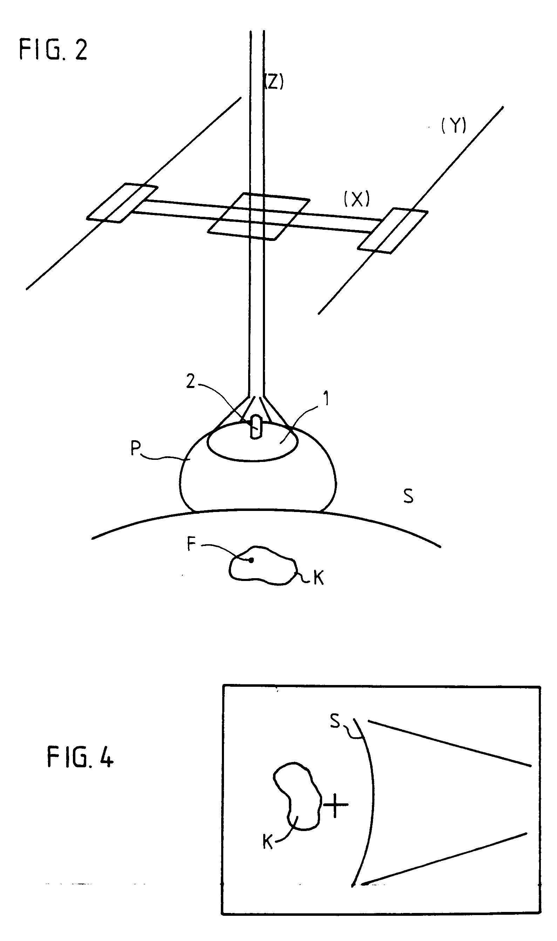

A la figure 2, on a représenté un transducteur principal 1 en forme de calotte sphérique supporté par un montage qui en permet les déplacements suivant trois axes orthogonaux X, Y et Z. Ce montage a été représenté de manière schématique, sa réalisation étant à la portée de l'homme du métier. Suivant l'axe de la calotte sphérique est disposé un transducteur auxiliaire 2 de forme générale cylindrique, qui passe à travers la calotte 1 et lui est fixé. Une poche d'eau P est interposée entre la calotte 1 et la surface S du corps du patient, celui-ci étant supposé allongé sur un plan horizontal.

La calotte 1 a par exemple 200 à 300 mm de diamètre et est composée d'un grand nombre (300 ou 400) d'éléments piézo-électriques 10, 11, etc... (figure 1) isolés les uns des autres et juxtaposés pour constituer une mosaïque. Ces éléments sont métallisés sur leurs deux faces, l'une des métallisations étant reliée à la masse et l'autre à des connexions d'excitation par un émetteur principal 3.

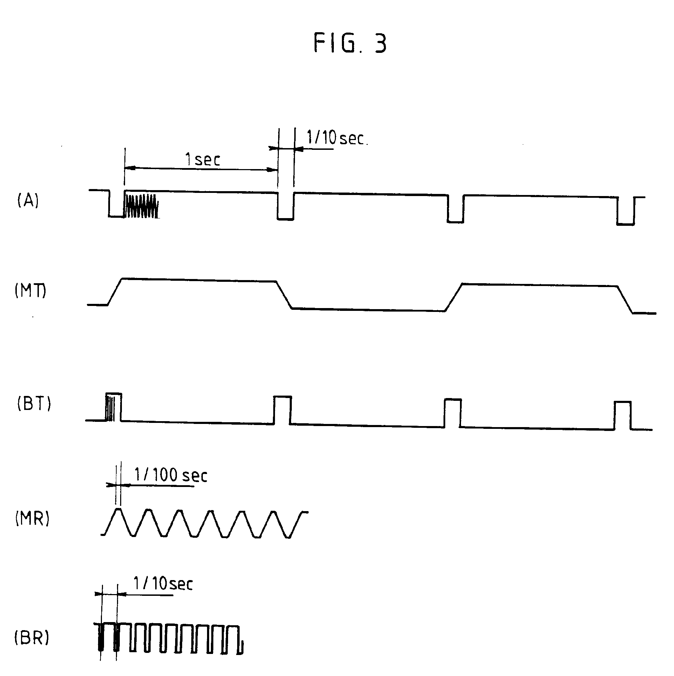

Ce dernier fournit un signal électrique A (figure 3) composé de trains d'ondes à haute fréquence (500 KHz par exemple) de puissance de crête relativement faible (une dizaine ou une centaine de watts par exemple), mais de durée relativement grande (par exemple de l'ordre de la seconde) séparés par des intervalles de temps de l'ordre de 1/10 sec., temps nécessaire pour la formation d'une image par le dispositif d'échographie. Il s'agit donc d'un régime d'émission quasi-continu, destiné au traitement. Un tel régime peut être obtenu au moyen d'émetteurs utilisant des transistors de puissance. De préférence, les éléments du transducteur 1 seront répartis en groupes excités chacun par un émetteur séparé (le rectangle 3 symbolisant l'ensemble de ces émetteurs), les éléments de chaque groupe étant répartis dans une même zone circulaire de la surface sphérique. En réglant les phases relatives des émissions, il est possible de modifier la répartition de l'énergie dans la région de focalisation du faisceau ultrasonore.

On a symbolisé par une entrée 31 de l'émetteur 3 un réglage de la puissance émise et par une entrée 32 un réglage de la durée des trains d'ondes. La tache focale formée au centre de la sphère pourra, avec cette technique, être très petite (diamètre de 2 ou 3 mm par exemple) et avoir une position rigoureusement fixe pour une position donnée du transducteur.

A la figure 1, on voit que le transducteur auxiliaire 2 est lui-même relié, d'une part, à un émetteur 21 d'impulsions électriques à haute fréquence, d'autre part, à un amplificateur de réception 22 suivi d'un convertisseur analogique-numérique 23, lui-même suivi d'une mémoire 24. L'émetteur 21 est synchronisé par un générateur d'impulsions 211 qui fournit 256 impulsions pendant chacun des intervalles de temps successifs de 1/10 de seconde. A chacun de ces intervalles de temps correspond un balayage complet d'un secteur angulaire prédéterminé θ (figure 1) par le faisceau émis par le transducteur 2, donc la formation, dans le plan de balayage, d'une image de la zone observée par le dispositif d'échographie.

Le transducteur 2 est avantageusement du type décrit dans les demandes de brevet français No 80 16717 déposée le 29 Juillet 1980 pour : "Sonde d'échographie à balayage sectoriel comportant deux liquides de couplage" et No 80 16718 déposée le 29 Juillet 1980 pour : "Sonde d'échographie à balayage sectoriel mécanique", c'est-à-dire qu'il comporte un élément piézo-électrique oscillant 200 commandé par un moteur 201, lui-même commandé par un circuit électronique que l'on a symbolisé par un rectangle 4. Ce circuit électronique fournit des signaux de commande du moteur 201 logé à l'intérieur du boîtier du transducteur 2 et est agencé de façon qu'une oscillation complète du moteur corresponde à la durée de formation d'une image définie ci-dessus (1/10 sec.).

Dans un premier mode de fonctionnement (traitement et contrôle) le commutateur 210 est en position I ainsi que les commutateurs 212 et 33.

Dans la position I des commutateurs 33 et 212, le générateur 211 est synchronisé par une première sortie 41 du circuit 4, et celui-ci est alors réglé, par des moyens non figurés, pour engendrer, sur sa sortie 43 reliée au moteur 201, des signaux ayant la forme d'onde (MT) représentée à la figure 4. Le balayage d'une image s'effectue donc en 1/10 s et est suivi d'un intervalle de temps de 1 s pendant lequel l'élément oscillant 200 reste immobile, si bien que le transducteur 2 ne reçoit pas d'échos.

Pendant les intervalles entre les périodes de balayage, un circuit 34 engendre des créneaux de 1 s qui servent à synchroniser l'émetteur 3, tandis que, pendant les périodes de balayage, un circuit 213 engendre des créneaux de 1/10 s qui servent à synchroniser le générateur 211.

Ainsi, dans ce mode de fonctionnement, le transducteur 1 engendre un faisceau d'ultrasons en régime quasi-continu, tandis que le dispositif d'échographie forme une image toutes les secondes dans les intervalles entre les trains d'ondes. On a représenté en (BT) la forme d'ondes des signaux alors émis par le générateur 211.

Dans un second mode de fonctionnement (repérage) le commutateur 210 restant en position I, le commutateur 33 est en position II, si bien que l'émetteur 3 n'est pas synchronisé et que le faisceau d'ultrasons focalisé n'est pas émis. Le commutateur 212 est également en position II, si bien que le générateur 211 est synchronisé par une deuxième sortie 42 du circuit 4 et celui-ci est réglé pour engendrer, sur sa sortie 43, des signaux ayant la forme d'onde (MR) représentée à la figure 3. Les balayages de 1/10 s sont donc séparés par des intervalles de temps de 1/100 sec. seulement et les images sont formées à partir des échos provenant de la réflexion des impulsions engendrées par le transducteur 2. Le générateur 211 fournit les signaux (BR).

Dans un troisième mode de fonctionnement (contrôle de la région focale), le commutateur 210 est en position III, si bien que l'émetteur 21 et le transducteur 2 n'émettent pas. Le commutateur 212 est encore en position II, si bien que le générateur 211 est synchronisé par la sortie 42 du circuit 4, et ce dernier est réglé comme dans le second mode de fonctionnement, si bien que les balayages de 1/10 s sont encore séparés par des intervalles de 1/100 sec. Le commutateur 33 est en position III et, par conséquent, l'émetteur 3 est maintenant synchronisé par le générateur 211 qui fournit alors les signaux (BR).

Dans ce troisième mode de fonctionnement, le dispositif échographique est donc constitué par l'émetteur 3, le transducteur 1 fonctionnant à l'émission et le transducteur 2 fonctionnant à la réception. Il en résulte qu'une image de la zone de concentration de l'énergie dans la région focale émise par le transducteur 1 est obtenue.

Les signaux échographiques reçus en 22 dans le premier ou le troisième modes de fonctionnement sont, après conversion analogique-numérique en 23, stockés ligne par ligne dans la mémoire 24, un dispositif d'adressage d'écriture 25, commandé par le circuit 4, permettant de faire correspondre les angles respectifs de déviation du faisceau émis et/ou reçu par le transducteur 2 aux lignes respectives de la mémoire. Un dispositif 26 de lecture rapide de la mémoire excite les bobines de déviation en X et en Y d'un tube cathodique 28, donc l'électrode de commande de brillance reçoit le contenu correspondant de la mémoire 24, transformé en signal analogique par un convertisseur numérique-analogique 27.

La réalisation pratique de tous les circuits décrits et représentés est à la portée de l'homme de l'Art. Le circuit de commande 4 pourra par exemple comporter un monovibrateur fournissant des créneaux de durée réglable à 1/100 s ou 1 s. suivant le mode de fonctionnement et des circuits de génération de tensions croissantes et décroissantes de durée 1/10 s., déclenchés par lesdits créneaux.

L'appareil qui vient d'être décrit fonctionne de la manière suivante :

Dans le mode de fonctionnement en repérage, l'opérateur recherche et localise la zone à traiter. Le dispositif de visualisation est agencé, de manière connue en soi, pour matérialiser sur l'écran du tube cathodique (par exemple par une croix) la position théorique de la tache focale dans le plan de coupe représenté, plan qui passe par l'axe de symétrie du transducteur 1. (Il s'agit d'échographie du type B).

L'opérateur commence par déplacer le transducteur 1 en X, jusqu'à ce que la tumeur apparaisse nettement sur l'écran, puis il le déplace en Y et Z, jusqu'à ce que la croix coïncide avec la région centrale de l'image de la tumeur (K, figure 4). A ce moment, les commutateurs peuvent être mis en position de contrôle de la région focale : seule celle-ci est alors rendue visible sur l'écran, avec une luminosité proportionnelle à la concentration d'énergie correspondante. On a ainsi une représentation de ce que sera la répartition de l'énergie de l'onde de traitement, ce qui permet de contrôler et de parfaire les réglages.

Pendant le traitement, l'appareil ne fournit qu'une image par seconde, mais cette cadence est suffisante pour permettre une vérification quasi permanente de la position de la tache focale.

Il est clair que l'appareil décrit permet le contrôle de l'évolution de la tumeur après chaque séquence de traitement.

## Revendications

1. Appareil de traitement combinant un générateur d'un faisceau focalisé d'ultrasons comportant un émetteur principal (3) d'ondes électriques à haute fréquence et un transducteur piézo-électrique principal (1), à un dispositif d'échographie comprenant un générateur auxiliaire d'impulsions électriques à haute fréquence (21-211) associé à un transducteur piézo-électrique auxiliaire (2) générant un faisceau ultrasonore d'examen et à des moyens de formation d'images de la zone à traiter, caractérisé en ce que des moyens (200-201-4) sont prévus pour provoquer un balayage de formation d'images de la zone à traiter par ledit faisceau ultrasonore d'examen, en ce que des moyens de commutation (210 à 213 et 39) et de réglage permettent de provoquer l'émission dudit faisceau focalisé par le transducteur principal excité par l'émetteur principal sous la forme de trains d'ondes périodiquement séparés par des blancs pendant un mode de fonctionnement en traitement et contrôle au cours duquel lesdits moyens de commutation et de réglage provoquent l'émission du faisceau d'examen et la formation d'images échographiques pendant lesdits blancs, que le transducteur principal a une surface active focalisante ayant un axe de symétrie, le transducteur auxiliaire est solidarisé au transducteur principal et effectue ledit balayage de la zone à traiter dans un plan qui passe sensiblement par ledit axe de symétrie, que ledit dispositif d'échographie comprend des moyens de matérialiser par un repère, sur écran de visualisation, la position théorique du foyer de ladite surface focalisante et que des moyens sont prévus pour commander le déplacement de l'ensemble des deux transducteurs jusqu'à obtention de la coïncidence entre ledit repère et l'image échographique de la zone à traiter.

## Patentansprüche

1. Behandlungsgerät, welches kombiniert : den Generator eines fokussierten Ultraschallwellenstrahls, mit einem Hauptsender (3) für elektrische Hochfrequenzwellen und einem piezoelektrischen Haupttransduktor (1) ; eine Echographievorrichtung, mit einem Hilfsgenerator für elektrische Hochfrequenzimpulse (21 - 211), der einem piezoelektrischen Hilfstransduktor (2) zugeordnet ist, welcher einen Untersuchungs-Ultraschallwellenstrahl abgibt ; und Mittel zur Erzeugung von Bildern der zu behandelnden Zone,
dadurch gekennzeichnet, dass Mittel (200-201-4) vorgesehen sind, damit besagter Untersuchungs-Ultraschallwellenstrahl die zu behandelnde Zone überstreicht, um Bilder zu erzeugen, dass Schaltmittel (210 bis 213 und 39) und Einstellmittel die Abgabe des besagten fokussierten Strahls durch den Haupttransduktor bewirken, der vom Hauptsender erregt wird, in Form von Wellenzügen, die in bestimmten Abständen durch Leerintervalle getrennt sind, während das Gerät im Behandlungs- und Kontrollmodus arbeitet, im Laufe dessen besagte Schalt- und Einstellmittel die Abgabe des Untersuchungsstrahles bewirken und die Formung von echographischen Bildern während der besagten Leerintervalle, dass der Haupttransduktor eine aktive Fokussierfläche mit einer Symmetrieachse aufweist, der Hilfstransduktor am Haupttransduktor befestigt ist und die zu behandelnde Zone in einer Ebene überstreicht, welche im wesentlichen durch besagte Symmetrieachse verläuft, dass besagte Echographievorrichtung Mittel aufweist, um auf einem Anzeigeschirm die theoretische Position der besagten fokussierenden Fläche zu markieren und Mittel vorgesehen sind, um die Verschiebung der von den beiden Transduktoren gebildeten Einheit zu steuern, bis die Überdeckung der besagten Markierung und des echographischen Bildes der zu behandelnden Zone erreicht ist.

## Claims

1. A treatment apparatus, combining a generator of a focused ultrasonic beam, comprising a main high frequency electric wave emitter (3) and a main piezoelectric transducer (1) with an echography device comprising an auxiliary high frequency electric pulse generator (21-211) associated with an auxiliary piezoelectric transducer (2) generating an ultrasound examination beam and with means for forming images of the zone to be treated,
characterized in that means (200-201-4) are provided for causing the zone to be treated to be swept over by said ultrasonic examination beam, in that switching means (210 to 213 and 39) and adjustment means cause emission of said focused beam by the main transducer, energized by the main emitter in the form of wave trains periodically separated by blanks during treatment and checking operating conditions during which said switching and adjustment means cause emission of the examination beam and formation of echographic images during said blanks, that the main transducer has an active focusing surface with an axis of symmetry, the auxiliary transducer is fast with the main transducer and provides a sweep of said zone to be treated in a plane which passes substantially through said axis of symmetry, that said echography device comprises means for materializng by a mark on a display screen the theoretical position of said focusing surface and that means are provided for controlling a displacement of the assembly of the two transducers until coincidence of said mark and the echographic image of the zone to be treated is obtained.

FIG.1

FIG. 2

FIG. 4

FIG. 3

(A)

1 sec

1/10 sec.

(MT)

(BT)

(MR)

1/100 sec

(BR)

1/10 sec